**Europäisches Patentamt**

**European Patent Office**

**Office européen des brevets**

(19)

(11) Publication number : **0 242 946 B1**

# EUROPEAN PATENT SPECIFICATION

(12)

(45) Date of publication of patent specification :
05.04.95 Bulletin 95/14

(51) Int. Cl.$^6$ : **G01N 27/416, G01N 33/00**

(21) Application number : **87301222.3**

(22) Date of filing : **12.02.87**

(54) **Calibration of combined oxygen and combustibles analyses.**

(30) Priority : **21.04.86 US 854256**

(43) Date of publication of application :
**28.10.87 Bulletin 87/44**

(45) Publication of the grant of the patent :
**05.04.95 Bulletin 95/14**

(84) Designated Contracting States :
**DE ES FR GB IT**

(56) References cited :
EP-A- 0 089 630
DE-A- 2 736 445
DE-A- 3 030 448
US-A- 3 838 021
US-A- 3 924 442
US-A- 4 279 618

(73) Proprietor : **INTERNATIONAL CONTROL AUTOMATION FINANCE S.A.**
**16 Rue des Bains**
**Ville de Luxembourg (LU)**

(72) Inventor : **Woolbert, Gordon Davies**
**6858 Burgundy Avenue NW**
**North Canton Ohio 44720 (US)**
Inventor : **Jewett, Scotty Young**
**5839 Alberta Drive**
**Lyndhurst Ohio 44124 (US)**
Inventor : **Robertson, John Walter Jr.**
**11940 Summers**
**Chesterland Ohio 44026 (US)**

(74) Representative : **Cotter, Ivan John et al**
**D. YOUNG & CO.**
**21 New Fetter Lane**
**London EC4A 1DA (GB)**

## Description

This invention relates to the calibration of combined oxygen and combustibles analysers.

Many known volatile atmosphere processes, such as those carried out in boiler furnaces and coal pulverisers, require constant monitoring to detect potentially flammable or explosive conditions and alert an operator to such conditions.

Presently, several systems are being used to monitor potentially dangerous coal pulveriser atmospheres. Thermocouples and infra-red gas analysers are two systems used to monitor pulverisers. A shield is needed to protect these systems from the corrosive coal particles in the coal pulveriser. The shield reduces heat conduction to the thermocouple system, thereby reducing response time. Infra-red gas analysers need to condition the sample gas, also decreasing response time and sensitivity.

A more reliable method used to monitor volatile atmospheres in coal pulverisers is to provide signals indicative of either oxygen, CO, or both. A certain quantity of each of these elements in a volatile atmosphere becomes indicative of the presence of a hazardous condition. Combination oxygen/combustibles analysers such as the Model OL230 supplied by Bailey Controls Company, a division of The Babcock & Wilcox Company, are used to provide such signals or combinations of them.

These analysers must be calibrated frequently to make sure that they are providing an accurate measurement of the aforementioned elements in the volatile atmosphere. Presently, the calibration procedures for such analysers are not automated with the entire analyser control system.

The present method for calibrating these analysers is manual. An operator manually introduces a test gas into the analyser, maintaining the air pressure as if the analyser was in operation. While maintaining the test gas pressure, calibration potentiometers are manually adjusted and test voltage outputs are monitored by hand-held voltmeters until desired calibrated outputs are attained.

A known method of calibrating a combustibles signal consists of introducing a test gas into the analyser and maintaining the sample air pressure as in the manual calibration of the oxygen signal. Zero and span (maximum scale value) values from the test gas are adjusted by an operator. The incoming sample signal is then calibrated according to these adjusted values.

The known calibration systems for combination oxygen/combustibles analysers are also manual. This inhibits the calibration of a plurality of analysers by one operator. The oxygen calibration operation requires at least fifteen minutes of operator time. This manual operation introduces the possibility of operator error and does not allow for repeatability of zero and span values. Further, the calibration of both combustibles and oxygen signals is not coordinated and thus hinders a plurality of analysers from being calibrated concurrently.

United States Patent US-A-3 924 442 describes a system for continuously measuring volumetric concentrations of gas which is automatically self calibrating and will correct volumetric measurements to a predetermined reference level. The system generates signals in response to zero and span gas phases which signals are used to generate motor drive signals for resetting potentiometers controlling the gain of an analyser in order to adjust the output thereof.

Since, however, the oxygen signal can drift significantly out of calibration before an operator becomes aware of the need for recalibration, a significant error with the oxygen signal is generated by the drift before it is detected and corrected.

According to a first aspect of the invention there is provided an automatic, periodically calibrating system for continuous output of calibrated signals from a combined oxygen and combustibles analyser comprising:

a combined oxygen and combustibles analyser for sensing a level of oxygen and a level of combustibles in a volatile atmosphere and for producing a first sample signal indicative of the oxygen level and a second sample signal indicative of the combustibles level;

means for introducing zero and span calibration test gases into the analyser; and

means for periodically calibrating the analyser including:

(a) a data control unit for automatically controlling the calibration of the analyser, for presetting optimum zero and span values for oxygen and combustibles, and for presetting alarm limits for oxygen and combustibles;

(b) a timer unit connected to the data control unit for setting discrete time intervals to activate the means for introducing the zero and span calibration test gases into the analyser;

(c) a mechanical unit connected to the timer unit and to the analyser, for introducing zero and span calibration test gases into the analyser;

(d) means for calculating zero and span values for oxygen and combustibles while the calibration test gases are introduced into the analyser;

(e) means for comparing said calculated zero and span values for oxygen and combustibles to the preset alarm limits for oxygen and combustibles;

(f) means for activating an operator alarm if the zero and span values exceed the preset alarm limits for oxygen and combustibles;

(g) means for calculating oxygen and combustibles drift adjustments based upon the zero and span values for oxygen and combustibles obtained from the calibration test gases, and upon the preset zero and span values for oxygen and combustibles; and

(h) a memory unit, connected to the data control unit, and to the means set forth in (d), (e), (f) and (g) above, for storing the preset optimum zero and span values, and the alarm limits; and

means for applying the oxygen and combustibles drift adjustments concurrently to the first and second sample signals, according to a predetermined mathematical relationship, to obtain calibrated output signals indicative of the oxygen and combustibles levels in the volatile atmosphere.

According to a second aspect of the invention there is also provided a method of automatically and periodically calibrating output signals from a combined combustibles and oxygen analyser comprising the steps of:

providing a source of predetermined time sequenced control signals;

purging the analyser by backflushing atmospheric air through the analyser for a predetermined period in response to the control signals;

establishing a signal indicative of the end of the purge;

introducing a zero calibration test gas to the analyser in response to the signal indicative of the end of the purge and providing a signal indicative of the end of the zero calibration test gas introduction;

introducing a maximum scale value calibration test gas to the analyser in response to the signal indicative of the end of the zero calibration test gas introduction and providing a signal indicative of the end of the maximum scale value calibration test gas introduction; and

adjusting an output of the analyser to read zero and maximum scale upon the signal indicative of the end of the span gas introduction.

A preferred embodiment of the present invention described hereinbelow overcomes or at least alleviates the problems associated with the non-automated control systems for oxygen/combustibles sensors by providing an improved safety monitoring system for oxygen and combustibles analysers using automatic calibration. The preferred system coordinates an automatic periodic calibration system with a signal sensing and safety alarm system to provide an entire monitoring/alarm/calibration system for oxygen and combustibles analysers. The system calibrates both the oxygen and combustibles signals in a fully automated fashion. A calibration procedure is initiated automatically on a predetermined interval such as every twenty-four hours. The calibration is performed concurrently on the oxygen signal and the combustible signal. Further, an alarm function is set into operation whenever calibrated zero and span values go beyond a preset limit. The preferred system then ensures that an accurate, hands-off calibration will be periodically performed. The preferred system is an accurate and reliable automatic calibration system which periodically calibrates the oxygen signal of the oxygen/combustibles analyser and coordinates it into a total control system for monitor/alarm/calibration of an entire safety monitoring unit. Further, the preferred system automatically adjusts both the span and zero values of both the oxygen and combustibles signals to minimise the effect of drift on the current analyser signal.

The invention will now be further described, by way of illustrative and non-limiting example, with reference to the accompanying drawings, in which:

Figure 1 is a schematic drawing of an automated calibration control system for oxygen and combustibles analysers according to a preferred embodiment of the present invention;

Figure 2 is a schematic view of control logic for a data control unit and a mechanical unit shown in Figure 1;

Figure 3 is a logic diagram for a logic unit shown in Figure 1;

Figure 4 is a logic diagram for an oxygen signal unit shown in Figure 1;

Figure 5 is an oxygen calibration graph used by the oxygen signal unit of Figure 4;

Figure 6 is a logic diagram for a combustibles ($CO_e$) signal unit shown in Figure 1; and

Figure 7 is a combustibles calibration graph used by the combustibles signal unit of Figure 6.

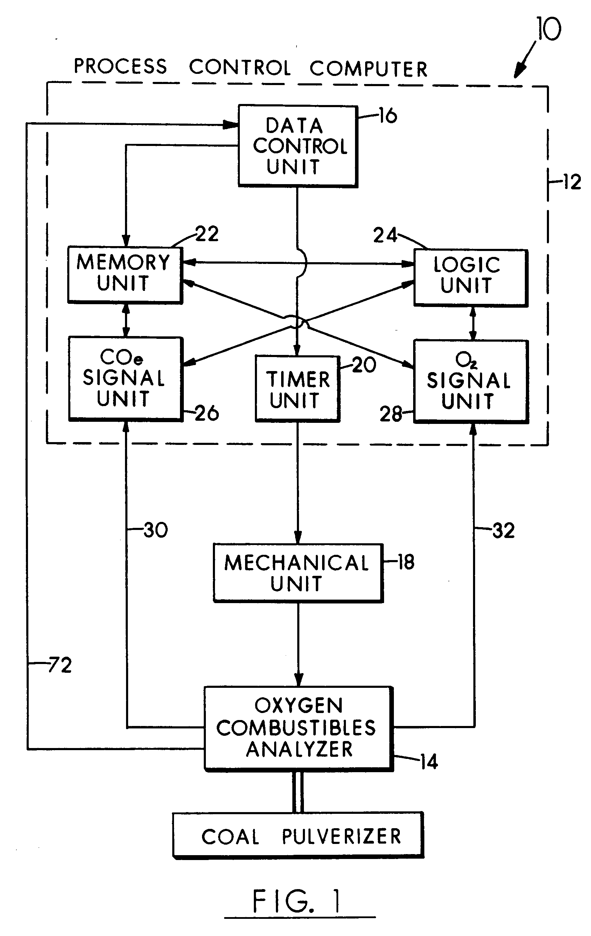

Referring now to the drawings generally, with particular reference to Figure 1, an automatic calibration system or calibration control system 10 includes a known process control computer 12, such as a Bailey Controls Company Model Network 90, and a known oxygen/combustibles analyser 14, such as a Bailey Controls Company Model OL230 gas analyser. All operations and calculations performed by the computer 12 use known hardware/firmward algorithms. These algorithms are called function block units or function blocks because each algorithm performs a specific function. The hardware/firmware used by the function blocks includes combinations of microprocessors with internal, unchangeable operation commands to perform the desired function and will be referred to as units.

More particularly, the system 10 includes a data control unit 16 which energises a mechanical unit 18, by way of a timer unit 20, allowing a manual input of data to be microprocessor memory unit 22. The mechanical unit 18 allows calibration gases to be introduced into the analyser 14. The memory unit 22 provides storage for and access to all necessary values used by the entire system 10. The timer unit 20 is an internal digital clock circuit which controls all timing functions needed by the system 10. A logic unit 24 receives inputs from the memory unit 22 and alerts an operator if the inputs go beyond preset limits for zero and maximum scale (hereafter referred to as span) and performs calculations needed by a combustibles (hereafter referred to as $CO_e$) signal unit 26 and an oxygen (hereafter referred to as $0_2$) signal unit 28. The signal units 26 and 28 respectively perform the actual calibration on a $CO_e$ indicating signal 30 and an $0_2$ indicating signal 32, originating from the oxygen/combustibles analyser 14.

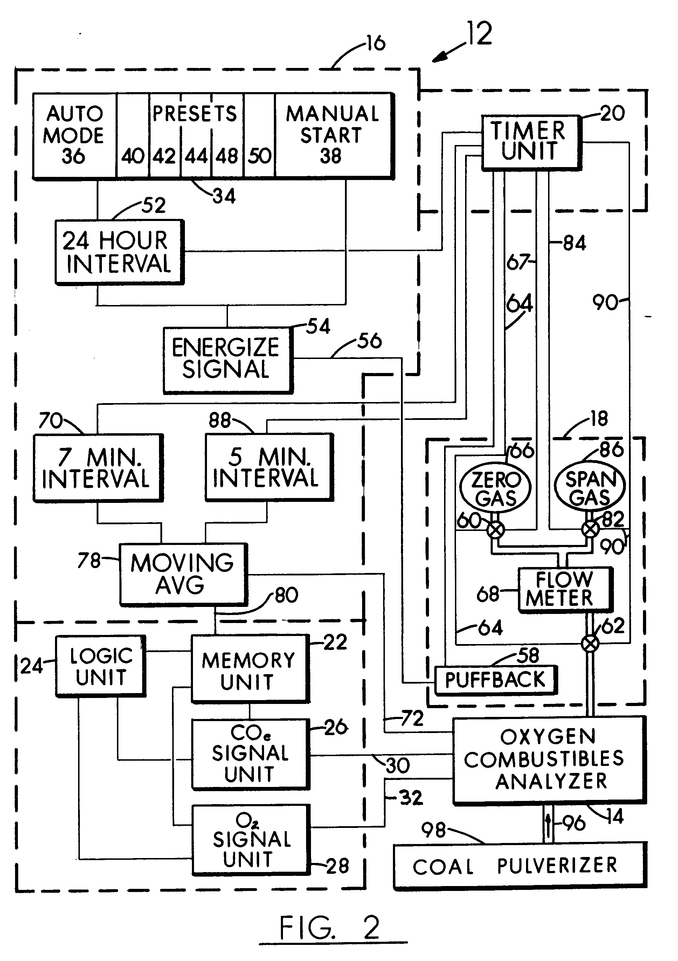

Referring now to Figure 2, the data control unit 16 has a manual data input station 34. The manual data input station 34 may be a known computerised data input keyboard or any other known manual data input apparatus. The manual data input station 34 allows an operator to selectively set the control unit 16 in an automatic mode 36, or to start a calibration sequence manually through a manual start button 38, to preset optimum zero and span values for $CO_e$ (40, 44) and $0_2$ (42, 48), respectively, and to preset alarm limits 50 for the aforementioned optimum zero and span values. The preset values 40, 42, 44, 48, 50 may be tuned or adjusted by the operator at any time through the manual data input station 34.

Usually, the control unit 16 will be set in the automatic mode 36. When in the automatic mode 36, the analyser 14 will be calibrated on a twenty-four hour interval 52. The interval will be maintained by the timer unit 20. The timer unit 20 provides that a calibration sequence will be peformed on the analyser 14 every day to ensure the accuracy of the oxygen and combustibles signals 30, 32 originating from the analyser 14. The manual start button 38 may also initiate a calibration sequence and will override the timer unit 20 to enable a calibration sequence to be performed at any time desired by the operator.

When a calibration sequence is initiated either by actuating the manual start button 38 or by the timer unit 20, an energise signal 54 is sent to the mechanical unit 18 along a line 56 from the process control computer 12. The mechanical unit 18 is physically attached by any known method to the analyser 14.

The mechanical unit 18 begins the calibration sequence by performing a cleaning operation 58 on the analyser 14. The cleaning operation 58 is called a "puffback". In this operation, the mechanical unit 18 forces air back through the analyser 14 to clean the analyser 14. The cleaning operation 58 is controlled by the timer unit 20 to continue for a period of ten seconds.

When the cleaning operation 58 is complete, known solenoid valves 60 and 62 were energised to open by a control signal 64 allowing a zero calibration gas 66 to flow through a flowmeter 68 into the analyser 14. The gas 66 flows through the analyser 14 for a seven minute interval 70 as controlled by the timer unit 20. The timer unit 20 sends a control signal 67 to deenergise the valve 60 when the seven minute interval 70 is over. The gas 66, used for zero calibration, usually consists of 1.0% $0_2$ and 0 ppm $CO_e$.

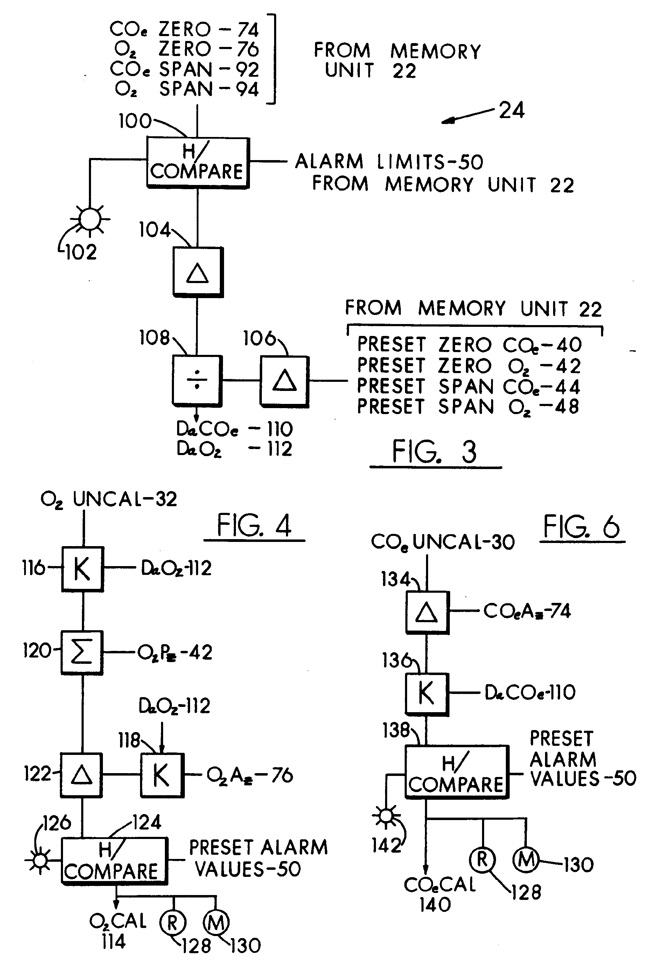

During the seven minute interval 70, zero value signals for $CO_e$ and $0_2$ as sensed by the analyser 14 are sent along a line 72 to the computer 12. When the seven minute interval 70 is over, signals 74, 76 (Figure 3) indicative of a one minute moving average of zero values for both $CO_e$ (74) and $0_2$ (76) are calculated by an internal averaging function block unit 78 of the computer 12. The signals 74 and 76 are then sent to the memory unit 22 along a line 80.

At the end of the seven minute interval 70, a third solenoid valve 82 is energised by a control signal 84 originating from the timer unit 20, allowing a span gas 86 to flow through the flowmeter 68 to the analyser 14. The span gas 86 usually contains 20.9% $0_2$ and 250 ppm $CO_e$. The span gas 86 flows through the analyser 14 for a five minute interval 88. At the end of the five minute interval 88 the timer unit 20 sends a control signal 90 to deenergise the valves 68 and 82.

During the five minute interval 88, span values for $CO_e$ and $0_2$ as sensed by the analyser 14 are sent to the computer 12 along the line 72. When the five minute interval 88 lapses, one minute moving averages of span values 92, 94 (Figure 3) for $CO_e$ (92) and $0_2$ (94) as sensed by the analyser 14 are calculated by the averaging function block unit 78 of the computer 12. The averages 92 and 94 are then sent to the memory unit 22 of the computer 12 along the line 80. The analyser 14 then returns to analysing a sample gas 96 from a coal pulveriser 98 and sending the signals indicative of $CO_e$ and $0_2$ content, 30 and 32 respectively, to the $CO_e$ signal unit 26 and the $0_2$ signal unit 28, respectively.

Referring now to Figure 3, when the data control unit 16 energises the mechanical unit 18, it also energises the memory unit 22. This allows the $CO_e$ and $0_2$ zero values, 74 and 76 respectively, and the $CO_e$ and $0_2$ span values, 92 and 94 respectively, to be stored in the memory unit 22.

When the memory unit 22 receives the zero values 74, 76 and the span values 92, 94, the logic unit 24 accesses them. The logic unit 24 first compares the values 74, 76, 92, 94 to the preset alarm limits 50 in a compare function block unit 100. If any of the zero or span values 74, 76, 92, 94 for $CO_e$ and $0_2$ are above their respective alarm limits 50, an alarm 102 will alert the operator to an inferior calibration condition and he

4

will take steps necessary to correct it, including but not limited to a manual adjustment of the analyser. These instances are rare due to daily calibration and the daily cleaning operation 58.

The logic unit 24 then performs a calculation using the values 74, 76, 92, 94; the preset optimum zero values for $CO_e$ and $O_2$, 40 and 42 respectively; and the preset span values for $CO_e$ and $O_2$, 44 and 48 respectively. The preset values 40, 42, 44, 48 are accessed from the memory unit 22. The calculations are performed in difference function block units 104 and 106 and a dividing function block unit 108. The same calculation is performed on both the $CO_e$ values 74, 92, 40, 44 and the $O_2$ values 76, 94, 42, 48 according to the following equation:

$$Da = \frac{P_s - P_z}{A_s - A_z}$$

where:

$Da$ = drift adjustment;
$P_s$ = preset span value ($CO_e$-44 $O_2$-48);
$P_z$ = preset zero value ($CO_e$-40 $O_2$-42);
$A_s$ = actual span value ($CO_e$-92 $O_2$-94); and
$A_z$ = actual zero value ($CO_e$-74 $O_2$-76).

The logic unit 24 sends calculated drift adjustments $DaCO_e$ 110 and $DaO_2$ 112 to the $CO_e$ signal unit 26 and the $O_2$ signal unit 28, respectively. The drift adjustments $DaCO_e$ 110 and $DaO_2$ 112 are drift adjustments to be applied to the $CO_e$ and $O_2$ sample signals 30 and 32, respectively. The drift adjustments 110 and 112 minimise the drift of the zero values 74, 76 and span values 92, 94 from their preset values 40, 42 and 44, 48, respectively.

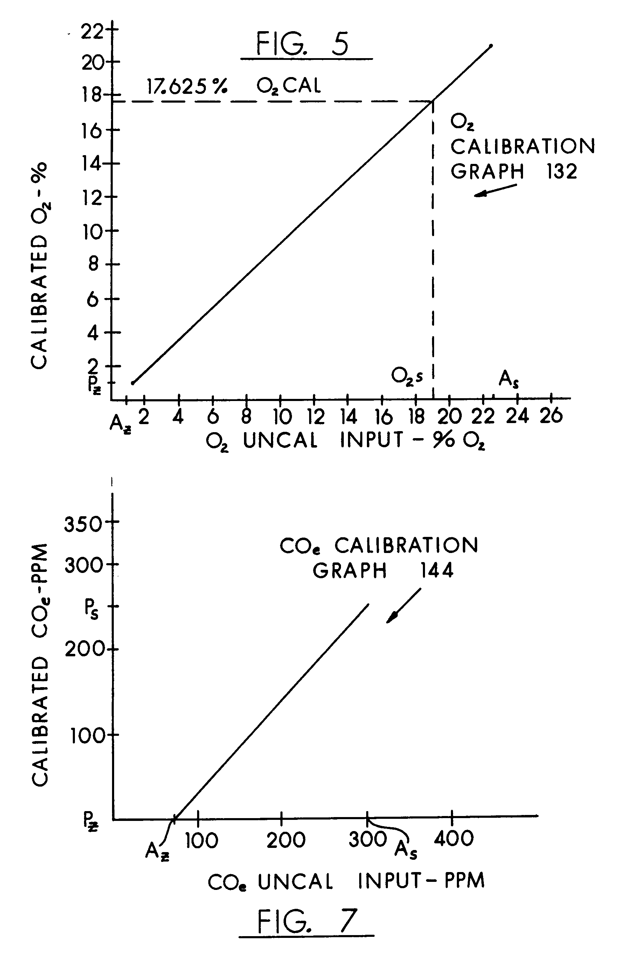

The $O_2$ signal unit 28, which may be embodied as shown in Figure 4, takes the $DaO_2$ calculation 112 from the logic unit 24, the $O_2P_z$ value 42 and the $O_2A_z$ value 76 from the memory unit 22, and the $O_2$ uncalibrated signal 32 from the analyser 14, and performs the following calculation on them:

$$O_2Cal = Da(O_2s) + P_z - Da(A_z)$$

where:

$O_2Cal$ = calibrated $O_2$ output 114;
$Da$ = drift adjustment 112;
$O_2s$ = $O_2$ sample signal 32;
$P_z$ = preset $O_2$ value 42; and
$A_z$ = actual $O_2$ zero value 76

The calculation to arrive at the calibrated $O_2$ output 114 is achieved by proportional function block units 116 and 118, a summation function block unit 120, and a difference function block unit 122.

The output 114 is continously monitored in a compare function block unit 124 to ensure that the coal pulveriser 98 being monitored does not go into a potentially dangerous situation. When the output 114 goes above its preset alarm value 50, the operator is alerted of the dangerous situation by an alarm 126 and can take necessary steps to stabilise the coal pulveriser 98.

The output 114 is sent to a known chart recorder 128 and also may be displayed on a known cathode ray tube monitor 130.

The $O_2$ signal unit 28 creates a calibration line graph 132, depicted in Figure 5, through its calculation. The $DaO_2$ value 112 is actually a proportion of error between the preset optimum scale and the scale the analyser 14 is outputting. The $DaO_2$ value 112 is then multiplied by the analyser $O_2$ output 32 to arrive at the calibrated $O_2$ output 114 with an offset added in for the preset zero value 42. The calibration graph 132 depicts the calibrated output 114, equal to 17.625% $O_2$ content, for the given uncalibrated analyser output 32, equal to 19% $O_2$. This calculation is performed continuously on the uncalibrated output 32 to give the continuous calibrated $O_2$ signal 114.

The $CO_e$ signal unit 26, which may be embodied as shown in Figure 6, operates in the same fashion as the $O_2$ signal unit 28. The $CO_e$ signal unit 26 uses the same calculation used in the $O_2$ signal unit 28, but performs this calculation using the $CO_e$ values 30, 74, 110. This calculation has no zero offset added in, however, because the preset zero value 28 is always zero. This calculation is performed in a difference function block unit 134 and a proportional function block unit 136. The $CO_e$ signal unit also uses a compare function block unit 138 to monitor a calibrated $CO_e$ signal 140 and alert the operator to a dangerous condition by an alarm 142. The calibrated $CO_e$ signal 140 may also be displayed on the cathode ray tube monitor 130 or the recorder 128.

A $CO_e$ calibration graph 144 is shown in Figure 7.

## Claims

1. An automatic, periodically calibrating system for continuous output of calibrated signals from a combined oxygen and combustibles analyser comprising:

   a combined oxygen and combustibles analyser (14) for sensing a level of oxygen and a level of combustibles in a volatile atmosphere and for producing a first sample signal indicative of the oxygen level and a second sample signal indicative of the combustibles level;

   means (18) for introducing zero and span calibration test gases into the analyser;

   means for periodically calibrating the analyser including:

   (a) a data control unit (16) for automatically controlling the calibration of the analyser, for presetting optimum zero and span values for oxygen and combustibles, and for presetting alarm limits for oxygen and combustibles;

   (b) a timer unit (20) connected to the data control unit for setting discrete time intervals to activate the means for introducing the zero and span calibration test gases into the analyser;

   (c) a mechanical unit (18) connected to the timer unit and to the analyser, for introducing zero and span calibration test gases into the analyser;

   (d) means (24, 26, 28) for calculating zero and span values for oxygen and combustibles while the calibration test gases are introduced into the analyser;

   (e) means (24) for comparing said calculated zero and span values for oxygen and combustibles to the preset alarm limits for oxygen and combustibles;

   (f) means (24) for activating an operator alarm if the zero and span values exceed the preset alarm limits for oxygen and combustibles;

   (g) means (24) for calculating oxygen and combustibles drift adjustments based upon the zero and span values for oxygen and combustibles obtained from the calibration test gases, and upon the preset zero and span values for oxygen and combustibles; and

   (h) a memory unit (22), connected to the data control unit, and to the means set forth in (d), (e), (f) and (g) above, for storing the preset optimum zero and span values, and the alarm limits; and

   means (24, 26, 28) for applying the oxygen and combustibles drift adjustments concurrently to the first and second sample signals, according to a predetermined mathematical relationship, to obtain calibrated output signals indicative of the oxygen and combustibles levels in the volatile atmosphere.

2. A system according to claim 1, wherein the means for calculating zero and span values for oxygen and combustibles while the calibration test gases are introduced into the analyser comprises: an oxygen signal unit (28) for calculating a calibrated output signal indicative of the oxygen level in the calibration test gases and outputting the same, a combustibles signal unit (26) for calculating a calibrated output signal indicative of the combustibles level in the calibration test gases and outputting the same, and a logic unit (24) connected to said oxygen signal unit and to said combustibles signal unit.

3. A system according to claim 2 wherein the oxygen signal unit (28) and the combustibles signal unit (26) each further comprise a comparing unit (124, 138) for comparing the calibrated output signals indicative of the oxygen and combustibles levels to the preset alarm limits for oxygen and combustibles, respectively, and alarming means for alerting an operator whenever either of the comparing units indicates that the calibrated output signals indicative of the oxygen and combustibles levels are above the preset limits.

4. A system according to claim 1, wherein the data control unit has manual input means for inputting the optimum preset zero and span values of oxygen and combustibles and a manual override capability to allow an operator to commence a calibration sequence.

5. A system according to claim 1 wherein the means for calculating the oxygen and combustible signals drift adjustments applies the following mathematical relationships:

$$DaCO_e = \frac{P_sCO_e - P_zCO_e}{A_sCO_e - A_zCO_e}; \qquad D_aO_2 = \frac{P_sO_2 - P_zO_2}{A_sO_2 - A_zO_2}$$

where:

$D_aCO_e$ = Drift Adjustment for Combustibles ($CO_e$)

$D_aO_2$ = Drift Adjustment for Oxygen ($O_2$)

$P_sCO_e$ = Preset Span Valve (for $CO_e$)

$P_sO_2$ = Preset Span Valve (for $O_2$)

$P_zCO_e$ = Preset Zero Valve (for $CO_e$)

$P_z0_2$ = Preset Zero Valve (for $0_2$)
$A_sC0_e$ = Actual Span Valve (for $C0_e$)
$A_s0_2$ = Actual Span Valve (for $0_2$)
$A_zC0_e$ = Actual Zero Valve (for $C0_e$)
$A_z0_2$ = Actual Zero Valve (for $0_2$).

6. A system according to claim 5 wherein the oxygen and combustibles drift adjustments are applied to the first and second sample signals according to the following mathematical relationships:

$$0_2 \text{ Cal } = D_a0_2 \times (0_2s) + P_z0_2 - D_a0_2 \times (A_z0_2)$$
$$C0_e \text{ Cal } = D_aC0_e(C0_es) - D_aC0_e \times (A_zC0_e)$$

where:
$0_2$ Cal = calibrated $0_2$ output
$C0_e$ Cal = calibrated $C0_e$ output
$D_aC0_e$ = Drift adjustment to $C0_e$
$D_a0_2$ = Drift adjustment to $0_2$
$C0_es$ = $C0_e$ sample signal
$0_2s$ = $0_2$ sample signal
$P_z0_2$ = Preset $0_2$ zero valve
$A_zC0_e$ = Actual $C0_e$ zero valve
$A_z0_2$ = Actual $0_2$ zero valve.

7. A system according to claim 1 wherein the means for periodically calibrating the analyser further comprises a puffback cleaning unit for performing a cleaning operation in the analyser for a duration long enough to purge said analyser before introducing the zero and span calibration test gases into the analyser.

8. A method of automatically and periodically calibrating output signals from a combined combustibles and oxygen analyser comprising the steps of:
   providing a source of predetermined time sequenced control signals;
   purging the analyser by backflushing atmospheric air through the analyser for a predetermined period in response to the control signals;
   establishing a signal indicative of the end of the purge;
   introducing a zero calibration test gas to the analyser in response to the signal indicative of the end of the purge and providing a signal indicative of the end of the zero calibration test gas introduction;
   introducing a maximum scale value calibration test gas to the analyser in response to the signal indicative of the end of the zero calibration test gas introduction and providing a signal indicative of the end of the maximum scale value calibration test gas introduction; and
   adjusting an output of the analyser to read zero and maximum scale upon the signal indicative of the end of the span gas introduction.

9. A method according to claim 8 wherein the zero calibration test gas is concurrently made up of a gas content of 0 ppm combustibles and 1.0% oxygen.

10. A method according to claim 8 wherein the maximum scale value calibration test gas is concurrently made up of a gas content of 250 ppm combustibles and 20.9% oxygen.

11. A method according to claim 8 wherein a first sample signal indicative of a level of oxygen in a volatile atmosphere and a second sample signal indicative of a level of combustibles in a volatile atmosphere are adjusted concurrently.

## Patentansprüche

1. Automatisches, periodisch eichendes System für kontinuierliche Abgabe geeichter Signale von einem kombinierten Sauerstoff- und Brennstoffanalysengerät mit:
   einem kombinierten Sauerstoff- und Brennstoffanalysengerät (14) zum Abfühlen eines Sauerstoffgehaltes und eines Brennstoffgehaltes in einer flüchtigen Atmosphäre und zur Erzeugung eines den Sauerstoffgehalt anzeigenden ersten Probensignals und eines den Brennstoffgehalt anzeigenden zweiten Probensignals,

einer Einrichtung (18) zur Einführung von Null- und Spannweiten-Eichtestgasen in das Analysengerät,

Einrichtungen zum periodischen Eichen des Analysengerätes mit:

(a) einer Datensteuereinheit (16) zum automatischen Steuern der Eichung des Analysengerätes, zur Voreinstellung optimaler Null- und Spannweitenwerte für Sauerstoff und Brennstoffe und zur Voreinstellung von Alarmgrenzen für Sauerstoff und Brennstoffe,

(b) einer Zeitgebereinheit (20), die mit der Datensteuereinheit verbunden ist, um einzelne Zeitintervalle einzustellen und so die Einrichtung zur Einführung der Null- und Spannweiten-Eichtestgase in das Analysengerät zu aktivieren,

(c) einer mechanischen Einheit (18), die mit der Zeitgebereinheit und dem Analysengerät verbunden ist, um Null- und Spannweiten-Eichtestgase in das Analysengerät einzuführen,

(d) einer Einrichtung (24, 26, 28) zur Berechnung von Null- und Spannweitenwerten für Sauerstoff und Brennstoffe, während die Eichtestgase in das Analysengerät eingeführt werden,

(e) einer Einrichtung (24) zum Vergleichen dieser berechneten Null- und Spannweitenwerte für Sauerstoff und Brennstoffe mit den voreingestellten Alarmgrenzen für Sauerstoff und Brennstoffe,

(f) einer Einrichtung (24) zum Aktivieren eines Personalalarms, wenn die Null- und Spannweitenwerte die voreingestellten Alarmgrenzen für Sauerstoff und Brennstoffe überschreibt,

(g) einer Einrichtung (24) zur Berechnung von Sauerstoff- und Brennstoffabweicheinstellungen auf der Basis der Null- und Spannweitenwerte für Sauerstoff und Brennstoffe, die man von den Eichtestgasen erhält, und auf der Basis der voreingestellten Null- und Spannweitenwerte für Sauerstoff und Brennstoffe und

(h) einer Speichereinheit (22), die mit der Datensteuereinheit und mit den in (d), (e), (f) und

(g) oben angegebenen Einrichtungen verbunden ist, um die voreingestellten optimalen Null- und Spannweitenwerte und die Alarmgrenzen zu speichern, und

einer Einrichtung (24, 26, 28) zur Anbringung der Sauerstoff- und Brennstoffabweicheinstellungen gleichzeitig an den ersten und zweiten Probesignalen gemäß einer vorbestimmten mathematischen Beziehung, um die Sauerstoff- und Brennstoffgehalte in der flüchtigen Atmosphäre anzeigende geeichte Ausgangssignale zu erhalten.

2. System nach Anspruch 1, bei dem die Einrichtung zur Berechnung von Null- und Spannweitenwerten für Sauerstoff und Brennstoffe, während die Eichtestgase in die Analyseneinrichtung eingeführt werden, eine Sauerstoffsignaleinheit (28) zur Berechnung eines den Sauerstoffgehalt in den Eichtestgasen anzeigenden geeichten Ausgangssignals und zur Abgabe desselben, eine Brennstoffsignaleinheit (26) zur Berechnung eines die Brennstoffkonzentration in den Eichtestgasen anzeigenden geeichten Ausgangssignals und zur Abgabe desselben sowie eine Logikeinheit (24), die mit der Sauerstoffsignaleinheit und der Brennstoffsignaleinheit verbunden ist, umfaßt.

3. System nach Anspruch 2, bei dem die Sauerstoffsignaleinheit (28) und die Brennstoffsignaleinheit (26) jeweils weiterhin eine Vergleichseinheit (124, 138) zum Vergleichen der die Sauerstoff- und Brennstoffgehalte anzeigenden geeichten Ausgangssignale mit den voreingestellten Alarmgrenzen für Sauerstoff bzw. Brennstoffe sowie eine Alarmeinrichtung zum Alarmieren von Personal, wann immer eine der Vergleichseinheiten anzeigt, daß die die Sauerstoff- und Brennstoffgehalte anzeigenden geeichten Ausgangssignale oberhalb der voreingestellten Grenzen liegen, umfassen.

4. System nach Anspruch 1, bei dem die Datensteuereinheit eine manuelle Eingabeeinheit zum Eingeben der optimalen voreingestellten Null- und Spannweitenwerte von Sauerstoff und Brennstoffen und eine manuelle Überlagerungsfähigkeit hat, um es einem Betätiger zu erlauben, eine Eichsequenz zu beginnen.

5. System nach Anspruch 1, bei dem die Einrichtung zur Berechnung der Abweichungseinstellungen der Sauerstoff- und Brennstoffsignale die folgenden mathematischen Beziehungen anwendet:

$$DaCO_e \;=\; \frac{P_sCO_e - P_zCO_e}{A_sCO_e - A_zCO_e}; D_aO_2 \;=\; \frac{P_sO_2 - P_zO_2}{A_sO_2 - A_zO_2}$$

worin:

$D_aCO_e$ = Abweichungseinstellung für Brennstoffe ($CO_e$)
$D_aO_2$ = Abweichungseinstellung für Sauerstoff ($O_2$)
$P_sCO_e$ = voreingestellter Spannweitenwert (für $CO_e$)
$P_sO_2$ = voreingestellter Spannweitenwert (für $O_2$)
$P_zCO_e$ = voreingestellter Nullwert (für $CO_e$)

$P_zO_2$ = voreingestellter Nullwert (für $O_2$)

$A_sCO_e$ = tatsächlicher Spannweitenwert (für $CO_e$)

$A_sO_2$ = tatsächlicher Spannweitenwert (für $O_2$)

$A_zCO_e$ = tatsächlicher Nullwert (für $CO_e$)

$A_zO_2$ = tatsächlicher Nullwert (für $O_2$)

6. System nach Anspruch 5, bei dem die Abweichungseinstellungen für Sauerstoff und Brennstoff auf die ersten und zweiten Probensignale gemäß den folgenden mathematischen Beziehungen angewendet werden:

$$O_2 \, Cal \; = \; D_aO_2 \, x \, (O_2s) \; + \; P_zO_2 \; - \; D_aO_2 \, x \, (A_zO_2)$$
$$CO_e \, Cal \; = \; D_aCO_e(CO_es) \; - \; D_aCO_e \, x \, (A_zCO_e)$$

worin:

$O_2Cal$ = geeichter $O_2$-Ausgang

$CO_eCal$ = geeichter $CO_e$-Ausgang

$D_aCO_e$ = Abweichungseinstellung für $CO_e$

$D_aO_2$ = Abweichungseinstellung für $O_2$

$CO_es$ = $CO_e$-Probensignal

$O_2s$ = $O_2$-Probensignal

$P_zO_2$ = voreingestellter $O_2$-Nullwert

$A_zCO_e$ = tatsächlicher $CO_e$-Nullwert

$A_zO_2$ = tatsächlicher $O_2$Nullwert.

7. System nach Anspruch 1, bei dem die Einrichtung für periodische Eichung des Analysengerätes weiterhin eine Rückblasreinigungseinheit zur Durchführung einer Reinigungsarbeit in dem Analysengerät für eine genügend lange Dauer umfaßt, um das Analysengerät vor der Einführung der Null- und Spannweiteneichtestgase in das Analysengerät zu spülen.

8. Verfahren zur automatischen und periodischen Eichung von Ausgangssignalen von einem kombinierten Brennstoff- und Sauerstoffanalysengerät mit den Stufen, in denen man:

eine Quelle für vorbestimmte in Zeitsequenz geordnete Steuersignale vorsieht,

das Analysengerät durch Spülen mit atmosphärischer Luft durch das Analysengerät während einer vorbestimmten Zeitdauer in Reaktion auf die Steuersignale spült,

ein das Ende der Spülung anzeigendes Signal erzeugt,

ein Nulleichtestgas in das Analysengerät in Reaktion auf das das Ende der Spülung anzeigende Signal einführt und ein das Ende der Nulleichtestgaseinführung anzeigendes Signal liefert,

ein Skalenmaximumwerteichtestgas in das Analysengerät in Reaktion auf das das Ende der Nulleichtestgaseinführung anzeigende Signal einführt und ein das Ende der Skalenmaximalwerteichtestgaseinführung anzeigendes Signal liefert und

einen Ausgang des Analysengerätes einstellt, um die Null- und Maximumskala an dem das Ende der Spannweitengaseinführung anzeigenden Signal abzulesen.

9. Verfahren nach Anspruch 8, bei dem das Nulleichtestgas gleichzeitig einen Gasgehalt von 0 ppm Brennstoff und 1,0 % Sauerstoff bekommt.

10. Verfahren nach Anspruch 8, bei dem das Skalenmaximumwerteichtestgas gleichzeitig einen Gasgehalt von 250 ppm Brennstoff und 20,9 % Sauerstoff bekommt.

11. Verfahren nach Anspruch 8, bei dem ein den Sauerstoffgehalt in einer flüchtigen Atmosphäre anzeigendes erstes Probensignal und ein einen Brennstoffgehalt in einer flüchtigen Atmosphäre anzeigendes zweites Probensignal gleichzeitig eingestellt werden.

## Revendications

1. Système d'étalonnage automatique, périodique, pour une sortie continue de signaux calibrés provenant d'un analyseur combiné d'oxygène et de combustible comprenant :

un analyseur combiné d'oxygène et de combustible (14) pour détecter un niveau d'oxygène et un niveau de combustible dans une atmosphère volatile et pour produire un premier signal d'échantillon indicatif du niveau d'oxygène et un second signal d'échantillon indicatif du niveau de combustible ;

un moyen d'introduction (18) pour introduire dans l'analyseur des gaz d'essai d'étalonnage de zéro et d'étendue de mesure ;

un moyen d'étalonnage pour étalonner de façon périodique d'analyseur incluant :

(a) un module de commande de données (16) pour commander automatiquement l'étalonnage de l'analyseur, pour prérégler des valeurs optimales de zéro et d'étendue de mesure pour l'oxygène et pour le combustible, et pour prérégler des limites d'avertissement pour l'oxygène et pour le combustible ;

(b) un module horloge (20) connecté au module de commande de données pour fixer des intervalles de temps discrets pour activer le moyen d'introduction de gaz d'essai d'étalonnage de zéro et d'étendue de mesure dans l'analyseur ;

(c) un module mécanique (18) connecté au module horloge et à l'analyseur pour introduire dans l'analyseur des gaz d'essai d'étalonnage de zéro et d'étendue de mesure ;

(d) un moyen de calcul (24, 26, 28) pour calculer des valeurs de zéro et d'étendue de mesure pour l'oxygène et pour le combustible pendant que les gaz d'essai d'étalonnage sont présents dans l'analyseur ;

(e) un moyen de comparaison (24) pour comparer lesdites valeurs de zéro et d'étendue de mesure calculées pour l'oxygène et pour le combustible aux limites d'avertissement préréglées pour l'oxygène et pour le combustible ;

(f) un moyen d'activation (24) pour activer un avertissement d'opérateur si les valeurs de zéro et d'étendue de mesure dépassent les limites d'avertissement préréglées pour l'oxygène et pour le combustible ;

(g) un moyen de calcul (24) pour calculer des ajustements de dérive d'oxygène et de combustible en se basant sur les valeurs de zéro et d'étendue de mesure pour l'oxygène et pour le combustible obtenues à partir des gaz d'essai d'étalonnage, et sur les valeurs préréglées de zéro et d'étendue de mesure pour l'oxygène et pour le combustible ; et

(h) un module de mémoire (22), connecté au module de commande de données, et aux moyens indiqués ci-dessus en (d), (e), (f), et (g), pour mémoriser les valeurs de zéro et d'étendue de mesure optimales préréglées et les limites d'avertissement ; et

un moyen (24, 26, 28) pour appliquer les ajustements de dérive d'oxygène et de combustible concurremment aux premier et second signaux d'échantillon, en fonction d'une relation mathématique prédéterminée, pour obtenir des signaux de sortie étalonnés indicatifs des niveaux d'oxygène et de combustible dans l'atmosphère volatile.

2. Système selon la revendication 1, dans lequel le moyen de calcul pour calculer les valeurs de zéro et d'étendue de mesure pour l'oxygène et le combustible pendant que les gaz d'essai d'étalonnage sont présents dans l'analyseur comprend : un module de signal d'oxygène (28) pour calculer un signal de sortie étalonné indicatif du niveau d'oxygène dans les gaz d'essai d'étalonnage et pour sortir celui-ci ; un module de signal de combustible (26) pour calculer un signal de sortie étalonné indicatif du niveau de combustible dans les gaz d'essai d'étalonnage et pour sortir celui-ci, et un module logique (24) connecté audit module de signal d'oxygène et audit module de signal de combustible.

3. Système selon la revendication 2, dans lequel le module de signal d'oxygène (28) et le module de signal de combustible (26) comprennent, en outre, chacun, un module de comparaison (124, 138) pour comparer les signaux de sortie étalonnés indicatifs des niveaux d'oxygène et de combustible aux limites d'avertissement préréglées pour, respectivement, l'oxygène et le combustible ; et moyen d'avertissement pour alerter un opérateur chaque fois que l'un ou l'autre des modules de comparaison indique que les signaux de sortie étalonnés indicatifs des niveaux d'oxygène et de combustible sont au-dessus des limites préréglées.

4. Système selon la revendication 1, dans lequel le module de commande de données possède un moyen d'entrée manuelle pour entrer les valeurs de zéro et d'étendue de mesure préréglées optimales d'oxygène et de combustible, et une possibilité de priorité manuelle pour permettre à un opérateur de lancer une séquence d'étalonnage.

5. Système selon la revendication 1, dans lequel le moyen de calcul de réglage de la dérive des signaux d'oxygène et de combustible applique les relations mathématiques suivantes :

$$DaCO_e = \frac{P_sCO_e - P_zCO_e}{A_sCO_e - A_zCO_e}; \qquad D_aO_2 = \frac{P_sO_2 - P_zO_2}{A_sO_2 - A_zO_2}$$

où :

$D_aCO_e$ est le réglage de dérive pour le combustible ($CO_e$) ;

$D_aO_2$ est le réglage de dérive pour l'oxygène ($O_2$) ;

$P_sCO_e$ est la valeur d'étendue de mesure préréglée (pour $CO_e$) ;

$P_sO_2$ est la valeur d'étendue de mesure préréglée (pour $O_2$) ;

$P_zCO_e$ est la valeur de zéro préréglée (pour $CO_e$) ;

$P_zO_2$ est la valeur de zéro préréglée (pour $O_2$) ;

$A_SCO_e$ est la valeur d'étendue de mesure réelle (pour $CO_e$) ;

$A_sO_2$ est la valeur détendue de mesure réelle (pour $O_2$) ;

$A_zCO_e$ est la valeur de zéro réelle (pour $CO_e$) ;

$A_zO_2$ est la valeur de zéro réelle (pour $O_2$).

6. Système selon la revendication 5, dans lequel les réglages de dérive d'oxygène et de combustible sont appliqués aux premier et second signaux d'échantillon en fonction des relations mathématiques suivantes :

$$O_2 \, Cal \, = \, D_aO_2 \times (O_2s) \, + \, P_zO_2 \, - \, D_aO_2 \times (A_zO_2)$$
$$CO_e \, Cal \, = \, D_aCO_e(CO_eS) \, - \, D_aCO_e \times (A_zCo_e)$$

où :

$O_2 \, Cal$ est la sortie d'$O_2$ étalonnée ;

$CO_e \, Cal$ est la sortie de $CO_e$ étalonnée ;

$D_aCO_e$ est le réglage de dérive pour $CO_e$ ;

$D_aO_2$ est le réglage de dérive pour $O_2$ ;

$CO_es$ est le signal d'échantillon de $CO_e$ ;

$O_2s$ est le signal d'échantillon d'$O_2$ ;

$P_zO_2$ est la valeur de zéro d'$O_2$ préréglée ;

$A_zCO_e$ est la valeur de zéro de $CO_e$ réelle ;

$A_zO_2$ est la valeur de zéro d'$O_2$ réelle.

7. Système selon la revendication 1, dans lequel le moyen d'étalonnage pour étalonner de façon périodique l'analyseur comprend en outre un module de nettoyage par chasse pour effectuer une opération de nettoyage dans l'analyseur pendant une durée suffisamment longue pour purger ledit analyseur avant d'introduire dans l'analyseur les gaz d'essai d'étalonnage de zéro et d'étendue de mesure.

8. Procédé d'étalonnage automatique et périodique de signaux de sortie d'un analyseur combiné de combustible et d'oxygène comprenant les étapes :

de mise en place d'une source de signaux de commande prédéterminés séquencés dans le temps ;

de purge de l'analyseur en faisant écouler en retour de l'air atmosphérique dans l'analyseur pendant une période prédéterminée en réponse à des signaux de commande ;

d'établissement d'un signal indicatif de la fin de la purge ;

d'introduction d'un gaz d'essai d'étalonnage de zéro dans l'analyseur en réponse au signal indicatif de la fin de la purge et de délivrance d'un signal indicatif de la fin de l'introduction du gaz d'essai d'étalonnage de zéro ;

d'introduction d'un&a+132Hgaz d'essai d'étalonnage de valeurs d'échelle maximale dans l'analyseur, en réponse au signal indicatif de la fin de l'introduction du gaz d'essai d'étalonnage de zéro, et de délivrance d'un signal indicatif de la fin de l'introduction du gaz d'essai d'étalonnage de valeur d'échelle maximale ; et

de réglage d'une sortie de l'analyseur pour lire le zéro et l'échelle maximale, après le signal indicatif de la fin de l'introduction de gaz d'essai d'étendue de mesure.

9. Procédé selon la revendication 8, dans lequel le gaz d'essai d'étalonnage de zéro est constitué concurremment d'une teneur de gaz de combustible de 0 ppm et d'oxygène de 1,0 %.

10. Procédé selon la revendication 8, dans lequel le gaz d'essai d'étalonnage de valeur d'échelle maximale est constitué concurremment d'une teneur de gaz de combustible de 250 ppm et d'oxygène de 20,9 %.

11. Procédé selon la revendication 8, dans lequel un premier signal d'échantillon indicatif d'un niveau d'oxygène dans une atmosphère volatile et un second signal d'échantillon indicatif d'un niveau de combustible dans une atmosphère volatile sont réglés concurremment.

PROCESS CONTROL COMPUTER

DATA CONTROL UNIT — 16

MEMORY UNIT — 22

LOGIC UNIT — 24

CO$_e$ SIGNAL UNIT — 26

TIMER UNIT — 20

O$_2$ SIGNAL UNIT — 28

— 12

— 30

— 32

— 72

MECHANICAL UNIT — 18

OXYGEN COMBUSTIBLES ANALYZER — 14

COAL PULVERIZER

FIG. 1

FIG. 2

EP 0 242 946 B1

CO_e ZERO − 74  
O_2 ZERO − 76  
CO_e SPAN − 92  
O_2 SPAN − 94  

FROM MEMORY UNIT 22

100

H/COMPARE — ALARM LIMITS−50 FROM MEMORY UNIT 22

102

104 △

FROM MEMORY UNIT 22

108 ÷   106 △

PRESET ZERO CO_e −40  
PRESET ZERO O_2 −42  
PRESET SPAN CO_e−44  
PRESET SPAN O_2 −48

D_a CO_e − 110  
D_a O_2 − 112

FIG. 3

O_2 UNCAL−32

FIG. 4

116 — K — D_a O_2-112

120 — Σ — O_2 P_z −42

D_a O_2-112

118

122 — △ — K — O_2 A_z − 76

126   124

H/COMPARE — PRESET ALARM VALUES−50

O_2 CAL 114   R 128   M 130

CO_e UNCAL−30

FIG. 6

134 △ — CO_e A_z-74

136 K — D_a CO_e-110

138

H/COMPARE — PRESET ALARM VALUES −50

142

CO_e CAL 140   R 128   M 130

14

FIG. 5

17.625% O₂ CAL

O₂ CALIBRATION GRAPH 132

CALIBRATED O₂ - %

O₂ UNCAL INPUT - % O₂

CO_e CALIBRATION GRAPH 144

CALIBRATED CO_e - PPM

CO_e UNCAL INPUT - PPM

FIG. 7

15